# FASCICULE DE BREVET EUROPEEN

(11) **EP 1 875 209 B1**
(45) Date de publication et mention de la délivrance du brevet: **12.03.2014**
(21) Numéro de dépôt: 06709135.5
(22) Date de dépôt: 20.01.2006
(51) Int. Cl.: G01N 21/64, A61B 1/06

(54) **Dispositif d`imagerie de fluorescence par reflexion a deux longueurs d`onde**
Fluoreszenzabbildungsvorrichtung mit Reflexion bei zwei Wellenlängen
Fluorescence imaging device with two wavelength reflection

(30) Priorité: 14.02.2005 FR 0501471
(43) Date de publication de la demande: 09.01.2008
(73) Titulaire: Commissariat à l'Énergie Atomique et aux Énergies Alternatives, 75015 Paris (FR)
(72) Inventeur: PELTIE, Philippe, F-38760 Saint Paul De Varces (FR); BERGER, Michel, F-38640 Claix (FR); SAUZE, Roland, F-38000 Grenoble (FR); BONNET, Stéphane, F-38170 Seyssinet (FR)
(74) Mandataire: Hecké, Gérard
(86) Numéro de dépôt international: PCT/FR2006/000131
(87) Numéro de publication internationale: WO 2006/087437

(56) Documents cités:
- US-A- 5 205 291
- US-A- 5 741 648
- US-A1- 2003 158 470
- TANHUANPAA K ET AL: "Fluorescence imaging of pyrene-labeled lipids in living cells" BIOCHIMICA ET BIOPHYSICA ACTA. MOLECULAR CELL RESEARCH, ELSEVIER SCIENCE PUBLISHERS, AMSTERDAM, NL, vol. 1497, no. 3, 20 septembre 2000 (2000-09-20), pages 308-320, XP004278179 ISSN: 0167-4889
- TROY T ET AL: "Quantitative comparison of the sensitivity of detection of fluorescent and bioluminescent reporters in animal models" MOLECULAR IMAGING MIT PRESS USA, vol. 3, no. 1, janvier 2004 (2004-01), pages 9-23, XP009049182 ISSN: 1535-3508
- SZOELLOESI J ET AL: "AUTOFLUORESCENCE CORRECTION FOR FLUORESCENCE IN SITU HYBRIDIZATION" CYTOMETRY, ALAN LISS, NEW YORK, US, vol. 20, 1995, pages 356-361, XP001074135 ISSN: 0196-4763
- WAN WADE K ET AL: "Measurement of drug distribution in vascular tissue using quantitative fluorescence microscopy" JOURNAL OF PHARMACEUTICAL SCIENCES, vol. 88, no. 8, août 1999 (1999-08), pages 822-829, XP002332297 ISSN: 0022-3549
- PATENT ABSTRACTS OF JAPAN vol. 2000, no. 13, 5 février 2001 (2001-02-05) & JP 2000 292353 A (FUJI PHOTO FILM CO LTD), 20 octobre 2000 (2000-10-20)
- PATENT ABSTRACTS OF JAPAN vol. 2000, no. 22, 9 mars 2001 (2001-03-09) & JP 2001 128927 A (FUJI PHOTO FILM CO LTD), 15 mai 2001 (2001-05-15)
- TASSETTI V ET AL: "In vivo laser-induced fluorescence imaging of a rat pancreatic cancer with pheophorbide-a" PHOTOCHEMISTRY AND PHOTOBIOLOGY, vol. 65, no. 6, 1997, pages 997-1006, XP009049184 ISSN: 0031-8655

## Description

### Domaine technique de l'invention

L'invention concerne un dispositif d'imagerie de fluorescence par réflexion comportant au moins une première source de lumière d'une première longueur d'onde correspondant sensiblement à une longueur d'onde d'excitation d'un fluorophore de marquage présentant une longueur d'onde d'émission principale, la longueur d'onde d'excitation et la longueur d'onde d'émission principale délimitant un intervalle prédéterminé, le dispositif comportant une caméra et au moins une seconde source de lumière d'une seconde longueur d'onde décalée par rapport à la première longueur d'onde, de manière à ce que la seconde longueur d'onde soit en dehors dudit intervalle prédéterminé.

### État de la technique

L'imagerie par réflexion à fluorescence (« FRI : Fluorescence Reflection imaging ») est une technique largement utilisée pour la fluorescence in vivo. Elle consiste à effectuer une imagerie de fluorescence de zones marquées d'un fluorophore couplé le plus souvent à un anticorps qui se fixe spécifiquement sur des tissus ou organes malades par exemple des tissus cancéreux. La technique FRI est également utilisée pour l'imagerie in vitro, par exemple pour la lecture de biopuces. Les domaines concernés sont aussi bien la biologie végétale qu'animale. Par exemple, la technique FRI peut être mise en oeuvre pour suivre la progression de virus marqués d'un fluorophore dans les plantes. Par ailleurs, des techniques utilisant plusieurs marqueurs ont été proposées.

Pour l'imagerie in vitro, par exemple la lecture de biopuces, les lecteurs traditionnels sont des microscopes à épi-illumination munis de caméras de type CCD et adaptés généralement à des champs de vision de faible taille de l'ordre de quelques millimètres carrés.

Pour l'imagerie in vivo, on trouve généralement deux approches. Une première approche, typiquement utilisée pour l'imagerie du petit animal (souris, rat, etc...), consiste à utiliser une caméra de type CCD munie d'un dispositif annexe d'éclairage, par exemple d'éclairage annulaire ou d'éclairage sous incidence. Les appareils correspondants sont généralement encombrants. Une deuxième approche, destinée à l'utilisation dans le corps humain, consiste à utiliser un endoscope à l'extrémité duquel sont connectés une caméra et un système d'éclairage. Les endoscopes sont généralement limités à des longueurs d'onde dans la gamme du visible, le proche ultraviolet et le proche infrarouge étant à leur limite de transmission.

Il existe de nombreux dispositifs qui permettent de visualiser un champ opératoire, en particulier des dispositifs utilisant différents bandes spectrales pour obtenir deux images qui sont superposées par la suite. Par exemple, une lumière d'excitation d'un fluorophore qui marque des tissus biologiques et une lumière blanche pour avoir une vision réaliste du champ opératoire. Les méthodes utilisées présentent généralement des problèmes de mise en forme du faisceau d'éclairage et d'auto-fluorescence.

Le document US2001/007920, par exemple, décrit un système d'endoscopie comportant une unité d'illumination comportant une source de lumière blanche et une source laser de lumière d'excitation utilisée pour exciter une image de fluorescence d'un corps vivant. Le système comporte, de plus, une unité de détection d'images de fluorescence, comportant un détecteur du type CCD, et une unité de détection d'images classiques, comportant un détecteur du type CCD. Les deux images sont superposées par l'intermédiaire de moyens de superposition. Les deux sources de lumière sont activées en alternance, provoquant chacune l'exposition du détecteur associé par la lumière correspondante.

Le document US6537211, par exemple, décrit un système d'imagerie d'une surface de tissus cancéreux comportant une source de lumière blanche pour exciter une image de réflexion et une source de lumière UV pour exciter une image de fluorescence. Les deux images sont détectées alternativement par une caméra de type CCD commune et affichées sur un écran commun. La lumière d'excitation de fluorescence est bloquée lorsque la lumière blanche est utilisée pour l'illumination et vice versa.

Lorsqu'on détecte une cible marquée par un fluorophore, on est généralement gêné par l'autofluorescence des tissus et par la diffusion de lumière due aux tissus, en particulier lorsque les cibles sont disposées à une profondeur supérieure à 1 mm. Ces problèmes sont d'autant plus importants que la mesure est effectuée à des longueurs d'onde courtes, par exemple dans le bleu, et moins importants dans l'infrarouge. Cependant, dans l'infrarouge, le rendement des détecteurs est faible et le choix de fluorophores est restreint. Ainsi, on travaille essentiellement dans une bande d'excitation comprise entre 480nm et 780nm avec une bande d'émission comprise entre 520nm et 800nm. Par ailleurs, le signal parasite de diffusion et de l'autofluorescence n'est pas du tout stationnaire.

Le document US5741648 décrit une méthode d'analyse d'images de fluorescence de cellules dotées de marqueurs fluorescents. Le document décrit une technique pour déterminer l'autofluorescence d'un échantillon comportant les cellules marquées et illuminé avec une première longueur d'onde. Une seconde longueur d'onde d'excitation est choisie dans la queue du spectre d'excitation du marqueur fluorescent. L'irradiation avec la seconde longueur d'onde provoque essentiellement l'autofluorescence de l'échantillon, tandis que la première longueur d'onde provoque une forte excitation des marqueurs fluorescents. Des niveaux de gris correspondant à l'autofluorescence sont soustraits des niveaux de gris correspondant à l'excitation avec la première la longueur d'onde, afin de déterminer la fluorescence due aux marqueurs fluorescents. Le document décrit l'utilisation de filtres passe-bande pour la transmission des signaux de fluorescence et d'auto-fluorescence. Cependant, le résultat obtenu présente un bruit important.

### Objet de l'invention

L'invention a pour but de remédier à ces inconvénients et, en particulier, de permettre la localisation rapide et précise de cellules marquées dans des tissus biologiques et notamment de réduire le bruit de mesure.

Selon l'invention, ce but est atteint par les revendications annexées et, plus particulièrement, par le fait que, le décalage entre les première et seconde longueurs d'onde étant compris entre 30nm et 100nm, la caméra comporte des moyens de filtrage au moins des première et seconde longueurs d'onde, les moyens de filtrage étant transparents à la longueur d'onde d'émission principale et à des longueurs d'ondes sensiblement supérieures à la plus élevée des première et seconde longueurs d'onde, le dispositif comportant des moyens de synchronisation des première et seconde sources de lumière et de la caméra, pour activer alternativement l'une des première et seconde sources de lumière et faire acquérir à la caméra alternativement une image de fluorescence et une image de bruit de fond.

L'invention a également pour but un procédé de visualisation d'un objet utilisant un dispositif d'imagerie selon l'invention et comportant une étape de marquage de cellules par le fluorophore de marquage et alternativement une première étape d'activation de la première source de lumière et d'acquisition d'une image de fluorescence, et une seconde étape d'activation de la seconde source de lumière et d'acquisition d'une image du bruit de fond.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
Les figures 1 et 2 représentent respectivement une partie en vue de côté et une autre partie en vue de dessus d'un mode de réalisation particulier d'un dispositif selon l'invention.
La figure 3 représente la courbe de transmission d'un mode de réalisation particulier d'un filtre en fonction de la longueur d'onde λ.
La figure 4 représente une courbe de transmission d'un autre exemple.
La figure 5 illustre la synchronisation des temps d'activation de la première source de lumière, de la seconde source de lumière et de la caméra en fonction du temps.

### Description d'un mode particulier de réalisation

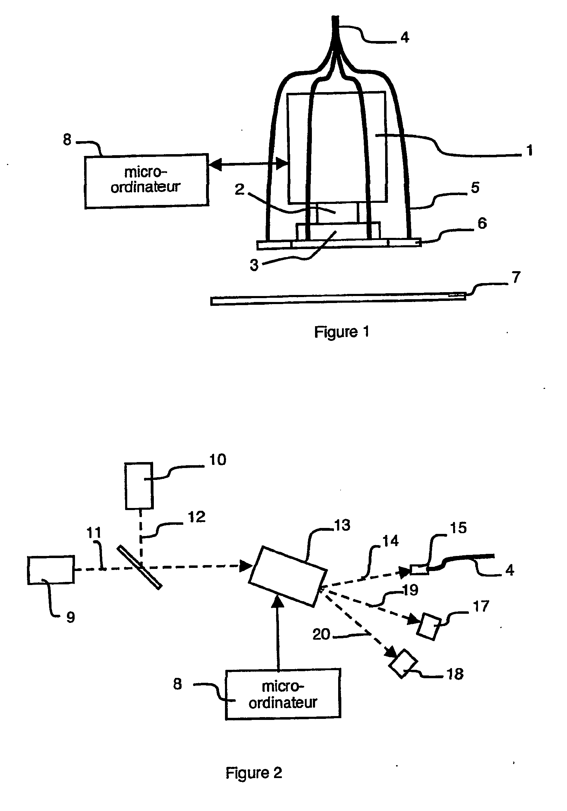

Sur la figure 1, le dispositif d'imagerie comporte une caméra 1 munie d'un objectif 2 et d'un filtre 3. Dans le mode de réalisation particulier représenté à la figure 1 de la lumière est amenée à la caméra 1 par une fibre optique principale 4 qui est reliée à une pluralité de fibres optiques 5 rattachées à un diffuseur 6 de lumière en forme d'anneau, permettant d'illuminer uniformément un objet disposé sur un support 7 d'objet, par exemple un échantillon biologique. L'objet peut également être un champ opératoire.

L'objet est marqué par un fluorophore de marquage. La caméra détecte la lumière émise par l'objet par l'intermédiaire du filtre 3 et transmet des signaux représentatifs d'images à une unité de traitement, par exemple un micro-ordinateur 8.

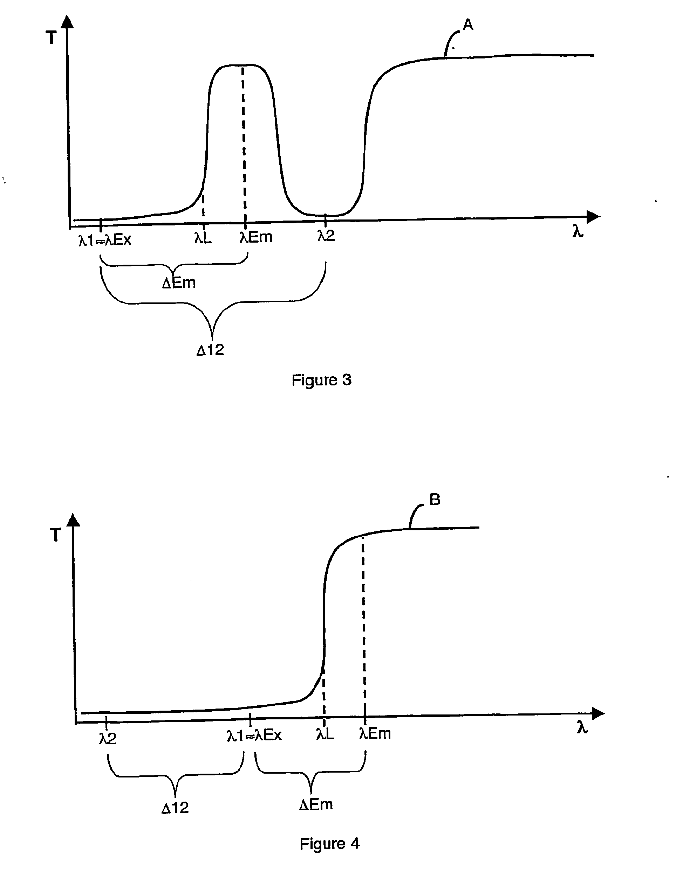

Sur la figure 2 sont représentées une première source de lumière 9 et une seconde source 10 de lumière. La première source de lumière 9 émet un premier faisceau 11 de lumière ayant une première longueur d'onde λ1 correspondant sensiblement à une longueur d'onde d'excitation λEx du fluorophore de marquage. Le fluorophore de marquage présente une longueur d'onde d'émission λEm principale. La longueur d'onde d'excitation λEx et la longueur d'onde d'émission λEm principale de fluorescence délimitent un intervalle ΔEm prédéterminé. La seconde source 10 de lumière produit un second faisceau 12 d'une seconde longueur d'onde λ2 décalée par rapport à la première longueur d'onde λ1. Le décalage Δ12 (figures 3 et 4) entre la seconde longueur d'onde λ2 et la première longueur d'onde λ1 est compris entre 30nm et 100nm. La seconde longueur d'onde λ2 est alors relativement proche de la première longueur d'onde λ1. Ainsi, la lumière de la seconde source 10 de lumière permet de provoquer l'autofluorescence de l'objet illuminé dans les mêmes conditions, quasiment, que la lumière de la première source 9 de lumière, sans pour autant provoquer la fluorescence du fluorophore de marquage, dont le décalage ΔEm est inférieur à 30 nm. Les deux longueurs d'onde provoquent donc quasiment le même signal d'autofluorescence, qui lui constitue un bruit de fond du signal de fluorescence du fluorophore de marquage.

Les premier et second faisceaux de lumière 11 et 12 sont dirigés, de préférence, sur l'entrée d'un filtre réglable acousto-optique 13 (« AOTF : Acousto-Optic Tunable Filter »), permettant de sélectionner la longueur d'onde d'un faisceau de sélection 14 orienté vers une entrée 15 de la fibre optique 4. Ainsi, le faisceau de sélection 14 a une longueur d'onde bien déterminée, notamment soit la première longueur d'onde λ1, soit la seconde longueur d'onde λ2. Le filtre réglable acousto-optique 13 est contrôlé par une unité de contrôle, par exemple constitué par le micro-ordinateur 8 servant également au traitement d'image.

Sur la figure 2, des première et seconde photodiodes 17 et 18 permettent de détecter l'intensité de faisceaux de référence 19 et 20 correspondant respectivement aux première et seconde longueur d'onde λ1 et λ2. En effet, lorsque la longueur d'onde du faisceau de sélection 14 est la première longueur d'onde λ1, un premier faisceau de référence 19 correspondant à l'ordre zéro du filtre 13 AOTF est incident sur la première photodiode 17 et permet de déterminer l'intensité émise par la première source de lumière 9. Un second faisceau de référence 20 correspondant à l'ordre zéro de la seconde longueur d'onde λ2 est détecté par la seconde photodiode 18 et permet, ainsi, de déterminer l'intensité émise par la seconde source de lumière 10.

Dans le mode de réalisation particulier correspondant à la figure 3, la seconde longueur d'onde λ2 est supérieure à la première longueur d'onde λ1. La seconde longueur d'onde λ2 doit être en dehors de l'intervalle ΔEm prédéterminé, délimité par la longueur d'onde d'excitation λEx et la longueur d'onde d'émission λEm principale du fluorophore de marquage. Comme illustré par la courbe de transmission A du filtre 3 représentée à la figure 3, le filtre 3 est opaque aux première (λ1) et seconde (λ2) longueurs d'onde, ce qui permet de filtrer la rétro-diffusion de la lumière d'excitation. Le filtre 3 est transparent à la longueur d'onde d'émission λEm principale. Le filtre 3 doit être transparent à des longueurs d'ondes sensiblement supérieures à la plus élevée des première et seconde longueurs d'onde λ1 et λ2, c'est-à-dire à la seconde longueur d'onde λ2, dans le mode de réalisation correspondant à la figure 3. Les longueurs d'ondes supérieures à la plus élevée des première et seconde longueurs d'onde λ1 et λ2 correspondent notamment à des signaux parasites d'autofluorescence de l'objet.

A titre d'exemple, les première et seconde longueurs d'onde λ1 et λ2 peuvent être respectivement 488nm et 532nm (décalage Δ12=44nm) lorsque le fluorophore utilisé est de la fluorescéine, 540nm et 600nm (décalage Δ12=60nm) lorsque le fluorophore utilisé est du Cy3, et 633nm et 690nm (décalage Δ12=57nm) lorsque le fluorophore utilisé est du Cy5.

Le filtre 3 correspondant à la figure 3 comporte, de préférence, un filtre passe-haut, ayant une longueur d'onde limite λL (figure 3) disposée entre la première longueur d'onde λ1 et la longueur d'onde d'émission λEm principale. Lorsque le fluorophore utilisé est de la fluorescéine, la longueur d'onde limite λL est, par exemple 500nm. De plus, dans le mode de réalisation illustré à la figure 3, le filtre 3 comporte un filtre de type coupe-bande bloquant une bande spectrale étroite (par exemple un filtre holographique de type « notch »), correspondant notamment à la seconde longueur d'onde λ2. Le filtre de type coupe-bande est superposé au filtre passe-haut.

Les sources de lumière présentant toujours une certaine largeur d'émission, l'écart entre la première longueur d'onde λ1 et la longueur d'onde limite λL doit être sensiblement supérieure à la largeur d'émission de la première source de lumière 9 pour assurer que la lumière de diffusion correspondante soit filtrée par le filtre 3. Pour la même raison, la bande spectrale bloquée par le filtre de type coupe-bande doit être supérieure à la largeur d'émission de la seconde source de lumière 10.

Dans l'exemple correspondant à la figure 4, la seconde longueur d'onde λ2 est inférieure à la première longueur d'onde λ1. Comme dans le mode de réalisation décrit précédemment, la seconde longueur d'onde λ2 doit être en dehors de l'intervalle λEm prédéterminé, délimité par la longueur d'onde d'excitation λEx et la longueur d'onde d'émission λEm principale du fluorophore de marquage. Comme illustré par la courbe de transmission B du filtre 3 représentée à la figure 4, le filtre 3 est, comme précédemment, opaque aux première (λ1) et seconde (λ2) longueurs d'onde et transparent à la longueur d'onde d'émission λEm principale. Le filtre 3 est transparent à des longueurs d'ondes sensiblement supérieures à la plus élevée des première et seconde longueurs d'onde λ1 et λ2, c'est-à-dire à la première longueur d'onde λ1 dans l'exemple correspondant à la figure 4.

Le filtre 3 correspondant à la figure 4 comporte, de préférence, un filtre passe-haut ayant une longueur d'onde limite λL disposée entre la première longueur d'onde λ1 et la longueur d'onde d'émission λEm principale.

L'écart entre la première longueur d'onde λ1 et la longueur d'onde limite λL doit, comme précédemment, être sensiblement supérieure à la largeur d'émission de la première source de lumière 9 pour assurer que la lumière de diffusion correspondante soit filtré par le filtre 3.

Selon l'invention, les première (9) et seconde (10) sources de lumière et la caméra 1 sont synchronisés de manière à activer alternativement l'une des première (9) et seconde (10) sources de lumière et faire acquérir à la caméra 1 alternativement une image de fluorescence et une image du bruit de fond. L'image de fluorescence comporte le signal de fluorescence émis par le fluorophore de marquage, d'une part, et des signaux parasites dus à l'autofluorescence, d'autre part, tandis que l'image du bruit de fond comporte essentiellement des signaux dus à l'autofluorescence des tissus et aux fluorescences parasites (filtres, optiques diverses, support de l'objet lorsqu'il s'agit de lecture in vitro).

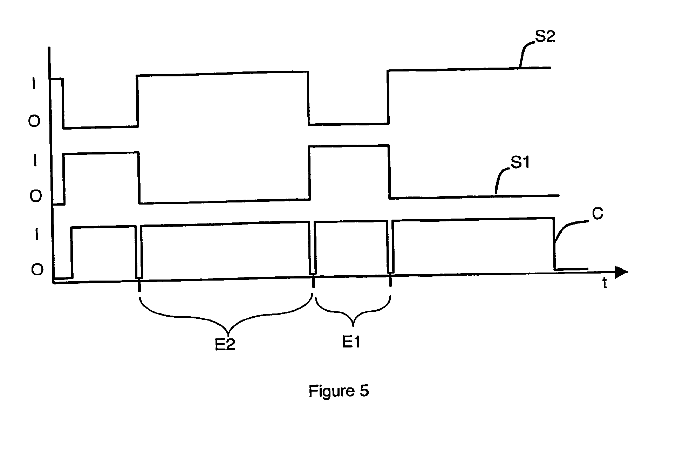

La synchronisation peut être effectuée par l'intermédiaire du micro-ordinateur 8 (figures 1 et 2) et du filtre réglable acousto-optique 13. Ainsi, comme représenté à la figure 5, la première source 9 de lumière est activée (la courbe S1 prend alors la valeur I à la figure 5) pendant que la seconde source 10 de lumière est désactivée (la courbe S2 prend alors la valeur O à la figure 5) et vice versa, tandis que la caméra 1 est activée (la courbe C prend la valeur I) respectivement pour l'acquisition d'images de chacune des sources. Entre deux acquisitions d'images, la caméra est désactivée (la courbe C prend la valeur O) pendant une durée très courte, de l'ordre de la microseconde.

Les temps d'intégration de chaque type d'image, image de fluorescence et image du bruit de fond, ne sont pas nécessairement les mêmes. La caméra peut être pilotée en temps d'intégration variable, soit de façon programmée, soit manuellement, par exemple, par le médecin. Par exemple, sur la figure 5, le temps d'illumination de la seconde source 10 de lumière (S2) est plus long que le temps d'illumination de la première source 9 de lumière (S1) et, ainsi, le temps d'intégration de la caméra associé à l'image du bruit de fond est plus long que le temps d'intégration associé à l'image de fluorescence.

L'image du bruit de fond et l'image de fluorescence peuvent, par exemple, être affichées simultanément sur un même écran. Toute combinaison de l'image du bruit de fond et de l'image de fluorescence peut également être déterminée et affichée, par exemple par l'intermédiaire de l'ordinateur 8. Dans un mode de réalisation particulier, l'image du bruit de fond est soustraite de l'image de fluorescence correspondante. Dans un autre mode de réalisation particulier, l'image de fluorescence est divisée par l'image du bruit de fond correspondante. Par ailleurs, l'image du bruit de fond permet de visualiser l'ensemble de l'objet illuminé, par exemple d'un champ opératoire.

Un procédé de visualisation d'un objet utilisant le dispositif selon l'invention comporte une étape de marquage de cellules par le fluorophore de marquage. Le procédé comporte ensuite, alternativement, une première étape E1 d'activation de la première source 9 de lumière et d'acquisition d'une image de fluorescence, et une seconde étape E2 d'activation de la seconde source 10 de lumière et d'acquisition d'une image du bruit de fond, comme illustré à la figure 5.

Même si la seconde longueur d'onde λ2 est relativement proche de la première longueur d'onde λ1, le signal d'autofluorescence des zones non-marquées par le fluorophore de marquage n'a pas forcément la même intensité. Dans un mode de réalisation particulier du procédé selon l'invention, on adapte des durées des première E1 et seconde E2 étapes, de manière à ce que une zone d'image correspondant à une zone prédéterminée non-marquée par le fluorophore de marquage présente une même luminosité sur une image de fluorescence et sur une image du bruit de fond.

Ainsi, lorsqu'on soustrait une image du bruit de fond d'une image de fluorescence, on obtient une image représentant essentiellement le signal dû au fluorophore de marquage. Lorsqu'on divise l'image de fluorescence par l'image du bruit de fond, on obtient une représentation accentuée des zones marquées par le fluorophore.

C'est donc notamment le signal d'autofluorescence à des longueurs d'ondes supérieures à la plus élevée des première et seconde longueurs d'onde λ1 et λ2 qui permet de caractériser le signal d'autofluorescence qui est détecté à la longueur d'onde d'émission λEm et qui ne peut pas être filtré sans diminuer le signal de fluorescence à la longueur d'onde d'émission λEm.

L'adaptation des durées des première E1 et seconde E2 étapes peut, par exemple, être effectuée par acquisition d'une image de fluorescence et d'une image du bruit de fond avec la même durée d'acquisition et calcul d'histogrammes de luminosité sur une portion d'image prédéterminée, correspondant à une zone non-marquée. Ensuite, les maximums des histogrammes permettent de déterminer automatiquement un rapport des durées des première E1 et seconde E2 étapes d'acquisition d'image, permettant d'obtenir la même luminosité sur une image de fluorescence et sur une image du bruit de fond.

On peut également adapter, par l'intermédiaire du filtre réglable acousto-optique 13, l'intensité du faisceau de sélection 14 pour chacune des première et seconde longueurs d'onde, de manière à ce que une zone d'image correspondant à une zone prédéterminée non-marquée par le fluorophore de marquage présente une même luminosité sur une image de fluorescence et sur une image du bruit de fond.

L'invention n'est pas limitée aux modes de réalisation représentés. En particulier, plusieurs premières et plusieurs secondes sources de lumière peuvent être utilisées, par exemple, quatre sources ayant respectivement des longueurs d'onde de 488nm et de 532nm, d'une part, et de 633nm et de 690nm, d'autre part, les fluorophores utilisés étant la fluorescéine et le Cy5. Ainsi, le filtre 3 est opaque à ces quatre longueurs d'onde et transparent aux deux longueurs d'onde d'émission des fluorophores et à des longueurs d'ondes sensiblement supérieures à la plus élevée des premières et secondes longueurs d'onde, c'est-à-dire au-dessus de 690nm.

## Revendications

1. Dispositif d'imagerie de fluorescence par réflexion comportant :
- une première source (9) de lumière d'une première longueur d'onde (λ1) correspondant sensiblement à une longueur d'onde d'excitation (λEx) d'un fluorophore de marquage présentant une longueur d'onde d'émission (λEm) principale, la longueur d'onde d'excitation (λEx) et la longueur d'onde d'émission (λEm) principale délimitant un intervalle (ΔEm) prédéterminé ;
- une seconde source (10) de lumière d'une seconde longueur d'onde (λ2) supérieure à la première longueur d'onde (λ1), la seconde longueur d'onde (λ2) étant en dehors dudit intervalle (ΔEm) prédéterminé pour générer une image de bruit de fond;
- une caméra (1), munie de moyens de filtrage (3) opaques aux première (λ1) et seconde (λ2) longueurs d'onde ;
dispositif **caractérisé en ce que** :
- le décalage (Δ12) entre les première (λ1) et seconde (λ2) longueurs d'onde étant compris entre 30nm et 100nm ;
- les moyens de filtrage (3) sont configurés de manière à être transparents à la longueur d'onde d'émission principale (λEm) et à des longueurs d'onde supérieures à la seconde longueur d'onde (λ2) ;
- le dispositif comporte des moyens de synchronisation (8, 13) des première (9) et seconde (10) sources de lumière et de la caméra (1), pour activer alternativement l'une des première (9) et seconde (10) sources de lumière et faire acquérir à la caméra (1) alternativement une image de fluorescence et une image de bruit de fond comprenant des signaux dus à l'autofluorescence.

2. Dispositif selon la revendication 1, **caractérisé en ce qu'**il comporte des moyens de soustraction (8) de l'image de fluorescence et de l'image du bruit de fond.

3. Dispositif selon la revendication 1, **caractérisé en ce qu'**il comporte des moyens de division (8) de l'image de fluorescence par l'image du bruit de fond.

4. Dispositif selon selon l'une quelconque des revendications 1 à 3, **caractérisé en ce que** les moyens de filtrage (3) comportent un filtre passe-haut ayant une longueur d'onde limite (λL) disposée entre la première longueur d'onde (λ1) et la longueur d'émission (λEm) principale, et un filtre de type coupe-bande correspondant à la seconde longueur d'onde (λ2).

5. Procédé de visualisation d'un objet utilisant un dispositif d'imagerie de fluorescence par réflexion selon l'une quelconque des revendications 1 à 4, **caractérisé en ce qu'**il comporte une étape de marquage de cellules par le fluorophore de marquage et alternativement une première étape (E1) d'activation de la première source (9) de lumière et d'acquisition d'une image de fluorescence, et une seconde étape (E2) d'activation de la seconde source (10) de lumière et d'acquisition d'une image du bruit de fond.

6. Procédé selon la revendication 5, **caractérisé en ce qu'**il comporte l'adaptation d'une image de fluorescence et d'une image du bruit de fond, de manière à ce qu'une zone d'image correspondant à une zone prédéterminée non-marquée par le fluorophore de marquage présente une même luminosité sur l'image de fluorescence et sur l'image du bruit de fond.

7. Procédé selon la revendication 6, **caractérisé en ce que** l'adaptation d'une image de fluorescence et d'une image du bruit de fond comporte l'adaptation de durées des première (E1) et seconde (E2) étapes.

8. Procédé selon l'une quelconque des revendications 5 à 7, **caractérisé en ce qu'**il comporte la soustraction de l'image de fluorescence et de l'image du bruit de fond.

9. Procédé selon l'une quelconque des revendications 5 à 7, **caractérisé en ce qu'**il comporte la division de l'image de fluorescence par l'image du bruit de fond.

## Patentansprüche

1. Vorrichtung zur Fluoreszenzabbildung mittels Reflexion, welche aufweist:
- eine erste Lichtquelle (9) von Licht mit einer ersten Wellenlänge (λ1), die im Wesentlichen einer Erregungswellenlänge (λEx) eines Markierungs-Fluorophors entspricht, welches eine Haupt-Strahlungswellenlänge ((λEm) aufweist, wobei die Erregungswellenlänge (λEx) und die Haupt-Strahlungswellenlänge ((λEm) ein vorbestimmtes Intervall (ΔEm) abgrenzen;
- eine zweite Lichtquelle (10) von Licht mit einer zweiten Wellenlänge (λ2), die größer ist als die erste Wellenlänge (λ1), wobei die zweite Wellenlänge (λ2) außerhalb des genannten vorbestimmten Intervalls (ΔEm) liegt, um ein Bild des Untergrundrauschens zu erzeugen,
- eine Kamera (1), die mit Filtermitteln (3) versehen ist, welche für die erste Wellenlänge (λ1) und die zweite Wellenlänge (λ2) undurchlässig sind,
**dadurch gekennzeichnet,**
**dass**
- die Verschiebung (Δ12) zwischen der ersten Wellenlänge (λ1) und der zweiten Wellenlänge (λ2), zwischen 30 nm und 100 nm beträgt,
- die Filtermittel (3) dergestalt konfiguriert sind, dass sie für die Haupt-Strahlungswellenlänge ((λEm) sowie für Wellenlängen, die größer sind als die zweite Wellenlänge (λ2), durchlässig sind;
- die Vorrichtung Mittel zur Synchronisierung (8, 13) der ersten und der zweiten Lichtquelle (9, 10) und der Kamera (1) aufweist, um eine der ersten und der zweiten Lichtquelle (9, 10) abwechselnd zu aktivieren und die Kamera (1) abwechselnd ein Fluoreszenzbild und ein Bild des Untergrundrauschens machen zu lassen, das durch Autofluoreszenz bedingte Signale beinhaltet.

2. Vorrichtung nach Anspruch 1,
**dadurch gekennzeichnet,**
**dass** sie Mittel zur Subtraktion (8) des Fluoreszenzbildes und des Bildes des Untergrundrauschens aufweist.

3. Vorrichtung nach Anspruch 1,
**dadurch gekennzeichnet,**
**dass** sie Mittel zur Teilung (8) des Fluoreszenzbildes durch das Bild des Untergrundrauschens aufweist.

4. Vorrichtung nach einem der Ansprüche 1 bis 3,
**dadurch gekennzeichnet,**
**dass** die Filtermittel (3) einen Hochpass-Filter mit einer Grenzwellenlänge (λL), die zwischen der ersten Wellenlänge (λ1) und der Haupt-Strahlungswellenlänge ((λEm) liegt, sowie einen Bandsperrenfilter, der der zweiten Wellenlänge (λ2) entspricht, umfassen.

5. Verfahren zur optischen Darstellung eines Objekts unter Verwendung einer Vorrichtung zur Fluoreszenzabbildung mittels Reflexion nach einem der Ansprüche 1 bis 4,
**dadurch gekennzeichnet,**
**dass** es einen Verfahrensschritt des Markierens von Zellen mittels des Markierungs-Fluorophors und wechselweise einen ersten Schritt (E1) des Aktivierens der ersten Lichtquelle (9) und Gewinnung eines Fluoreszenzbildes und einen zweiten Schritt (E2) des Aktivierens der zweiten Lichtquelle (10) und Gewinnung eines Bildes des Untergrundrauschens umfasst.

6. Verfahren nach Anspruch 5,
**dadurch gekennzeichnet,**
**dass** es die Anpassung eines Fluoreszenzbildes und eines Bildes des Untergrundrauschens umfasst, dergestalt, dass ein Bildbereich, der einem vorbestimmten, nicht mittels des Markierungs-Fluorophors markierten Bereich entspricht, sowohl in dem Fluoreszenzbild wie auch in dem Bild des Untergrundrauschens die gleiche Helligkeit aufweist.

7. Verfahren nach Anspruch 6,
**dadurch gekennzeichnet,**
**dass** die Anpassung eines Fluoreszenzbildes und eines Bildes des Untergrundrauschens die Anpassung der Zeitdauer des ersten Schritts (E1) und der des zweiten Schritts (E2) umfasst.

8. Verfahren nach einem der Ansprüche 5 bis 7,
**dadurch gekennzeichnet,**
**dass** es die Subtraktion des Fluoreszenzbildes und des Bildes des Untergrundrauschens umfasst.

9. Verfahren nach einem der Ansprüche 5 bis 7,
**dadurch gekennzeichnet,**
**dass** es die Teilung des Fluoreszenzbildes durch das Bild des Untergrundrauschens umfasst.

## Claims

1. Fluorescence reflection imaging device comprising
- a first light source (9) of a first wavelength (λ1) corresponding substantially to an excitation wavelength (λEx) of a marking fluorophore presenting a main emission wavelength (λEm), the excitation wavelength (λEx) and the main emission wavelength (λEm) delineating a predetermined interval (ΔEm),
- a second light source (10) of a second wavelength (λ2) higher than the first wavelength (λ1), the second wavelength (λ2) being outside said predetermined interval (ΔEm) for generating a background noise image ;
- a camera (1) provided with filtering means (3) opaque to the first and second wavelengths (λ1, λ2),
device **characterized in that** :
- the offset (Δ12) between the first (λ1) and second (λ2) wavelengths being comprised between 30nm and 100nm,
- the filtering means (3) are configured so as to be transparent at the main emission wavelength (λEm) and at wavelengths higher than the second (λ2) wavelength ;
- the device comprises synchronization means (8, 13) of the first (9) and second (10) light sources and of the camera (1) to alternately activate one of the first (9) and second (10) light sources and make the camera (1) alternately acquires a fluorescence image and a background noise image comprising signals coming from autofluorescence.

2. Device according to claim 1, **characterized in that** it comprises subtracting means (8) for subtracting the fluorescence image and the background noise image.

3. Device according to claim 1, **characterized in that** it comprises dividing means (8) for dividing the fluorescence image by the background noise image.

4. Device according to any one of claims 1 to 3, **characterized in that** the filtering means (3) comprise a high-pass filter having a limit wavelength (λL) located between the first wavelength (λ1) and the main emission wavelength (λEm), and a band-stop filter corresponding to the second wavelength (λ2).

5. Method for displaying an object using a fluorescence reflection imaging device according to any one of claims 1 to 4, **characterized in that** it comprises a step of marking cells by the marking fluorophore and alternately a first step (E1) of activation of the first light source (9) and acquisition of a fluorescence image, and a second step (E2) of activation of the second light source (10) and acquisition of a background noise image.

6. Method according to claim 5, **characterized in that** it comprises adjustment of a fluorescence image and of a background noise image so that an image area corresponding to a predetermined area not marked by the marking fluorophore presents the same luminosity on the fluorescence image and on the background noise image.

7. Method according to claim 6, **characterized in that** adjustment of a fluorescence image and of a background noise image comprises adjustment of the durations of the first (E1) and second (E2) steps.

8. Method according to any one of claims 5 to 7, **characterized in that** it comprises subtraction of the fluorescence image and of the background noise image.

9. Method according to any one of claims 5 to 7, **characterized in that** it comprises division of the fluorescence image by the background noise image.
